# EUROPEAN PATENT SPECIFICATION

(11) **EP 3 845 135 B1**
(45) Date of publication and mention of the grant of the patent: **22.03.2023**
(21) Application number: 18932712.5
(22) Date of filing: 13.11.2018
(51) Int. Cl.: A61B 17/34, A61B 1/00, A61B 17/00, A61B 17/16, A61B 17/17

(54) **CANNULA HAVING MULTIPLE OPERATING CHANNELS**
KANÜLE MIT MEHREREN ARBEITSKANÄLEN
CANULE AYANT DE MULTIPLES CANAUX DE FONCTIONNEMENT

(30) Priority: 05.09.2018 CN 201821449663 U
(43) Date of publication of application: 07.07.2021
(73) Proprietor: Dragon Crown Medical Co., Ltd., Jinan, Shandong 250101 (CN)
(72) Inventor: YANG, Wenzhou, Jinan, Shandong 250101 (CN); PAN, Huihui, Jinan, Shandong 250101 (CN)
(74) Representative: Sun, Yiming
(86) International application number: PCT/CN2018/000388
(87) International publication number: WO 2020/047688

(56) References cited:
- WO-A1-2017/109900
- CN-A- 1 861 013
- CN-A- 101 933 833
- CN-A- 102 319 090
- CN-A- 106 999 242
- CN-A- 108 309 395
- CN-Y- 201 160 881
- GB-A- 645 492
- US-A1- 2010 036 198
- US-A1- 2015 025 315
- US-A1- 2015 335 381
- US-A1- 2017 165 455

## Description

### TECHNICAL FIELD

This application relates to the technical field of medical device, and in particular to a multi-channel working cannula.

### BACKGROUND

Minimally invasive surgery refers to a surgery performed by use of modern medical instruments such as laparoscope or thoracoscope, as well as a minimally invasive channel device and related equipment. Since the minimally invasive surgery has the advantages of less trauma, light pain, and fast recovery, the concept of "minimally invasive" has penetrated into various fields of surgical operations.

In the prior art, the minimally invasive channel device includes a guide tube and an expansion tube, and the guide tube is arranged inside the expansion tube. In an actual surgery, when it is needed to grind the bones around the affected area, the abrasion drill needs to be inserted along the guide tube to the affected area for bone grinding. Because the expansion tube has only one guide tube, only the abrasion drill can be inserted at this time, the endoscope cannot be inserted at the same time, therefore, the abrasion drill is in a blind grinding state during the actual grinding process, so that the abrasion drill cannot always be accurately aligned with the part that needs to be ground during the bone grinding process; at the same time, after the end of the bone grinding, when the endoscope needs to be inserted for observation, it is needed to first remove the abrasion drill from the guide tube, and then insert the endoscope from the guide tube for observation. The operation procedure is cumbersome, which prolongs the operation time and causes a certain injury to the patients.

US 2015/335381 A1 refers to a device and method that provides a minimally-invasive approach to performing treatments on soft tissue, such as that found in the bladder. The device is useful for manipulating tissue such that treatment tools can be inserted into the tissue at a controlled depth.

GB 645 492 A refers to a surgical appliance comprising a cannula which is introduced into the body by means of a trocar or perforator.

CN 108309395 A discloses a working channel comprising a main working channel and an auxiliary working channel which are connected in parallel; a foramen intervertebrale endoscope is installed inside the main working channel, and a foramen intervertebrale molding instrument is installed in the auxiliary working channel.

### SUMMARY

This patent provides a multi-channel working cannula according to the enclosed claims to solve the problem of inconvenient observation of the working state of the endoscope in the prior art.

Compared with the prior art, the present invention has the following beneficial effects. The present invention includes a guide tube unit including a main guide tube and an accessory guide tube with the main guide tube and the accessory guide tubes integrally arranged; the length of the main guide tube is greater than that of the accessory guide tube, and the outlet end of the accessory guide tube is attached to the outer side wall of the main guide tube; an endoscope view hole is provided at the attachment site of the outlet end of the accessory guide tube on the outer side wall of the main guide tube, in an actual surgery process, when the device is working from the accessory guide tube, the endoscope can check the working status of the device through the endoscope view hole in the main guide tube; when the device is working from the main guide tube, the endoscope can also check the working status of the device in the accessory guide tube; because the center line of the main guide tube is not parallel with the center line of the accessory guide tube, and the outlet end of the accessory guide tube is attached to the outer side wall of the main guide tube, so that the inlet end of the main guide tube and the inlet end of the accessory guide tube are not adjacent, and the two inlet ends are separated by a certain distance, this can prevent the insertion of larger devices in the main guide tube from affecting the insertion of a device in the accessory guide tube. The working cannula has simple structure and convenient installation, making the operation more flexible and convenient.

### DESCRIPTION OF DRAWINGS

In order to explain the background technology or the technical solutions of the present invention more clearly, the accompanying drawings used in the prior art or in conjunction with the specific embodiments will be briefly introduced below. Obviously, the following accompanying drawings in conjunction with the specific embodiments are only used to facilitate the understanding of the embodiments of the present invention. For those of ordinary skill in the art, other accompanying drawings can also be obtained based on these accompanying drawings without paying creative work.
Figure 1 is a first schematic diagram of the axial side structure of a working cannula provided by an embodiment of the present invention;
Figure 2 is a schematic structural view of the guide plate provided by an embodiment of the present invention; and
Figure 3 is a second schematic diagram of the axial side structure of a working cannula provided by an embodiment of the present invention;

Reference numerals: 1: main guide tube, 2: accessory guide tube, 3: guide plate, 30: first through hole, 4: endoscope view hole, 5: first inclined opening, 6: second inclined opening, 7: connecting plate.

### Detailed Description

In order to enable those skilled in the art to better understand the technical solutions of the present invention, the technical solutions in the embodiments of the present invention will be clearly and completely described below in conjunction with the accompanying drawings in the embodiments of the present invention. Obviously, the embodiments described are merely some of, rather than all of the embodiments of the present invention. Based on the embodiments of the present invention, all other embodiments obtained by those of ordinary skill in the art without paying any creative effort shall fall within the protection scope of the present invention.

The multi-channel working cannula provided by the embodiment of the present invention includes a guide tube unit made of stainless steel, and the guide tube unit includes a main guide tube 1 and an accessory guide tube 2.

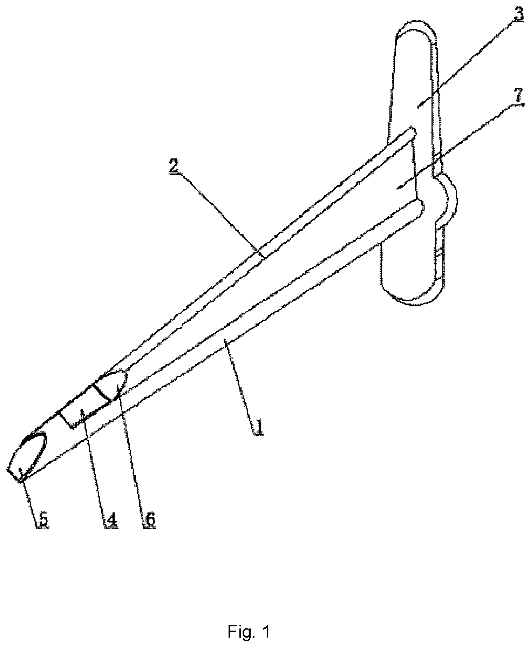

Figure 1 is a first schematic diagram of the axial side structure of a working cannula provided by an embodiment of the present invention. As shown in Figure 1, the main guide tube 1 and the accessory guide tube 2 are integrally arranged through a connecting plate 7. One end of the guide tube unit is the inlet end, and the other end is the outlet end.

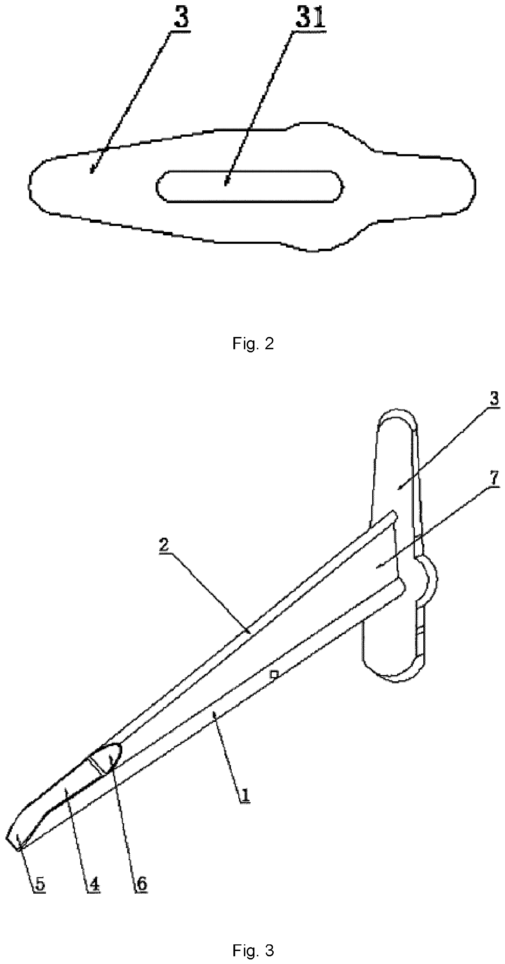

The inlet end of the guide tube unit is provided with a guide plate 3, Figure 2 is a schematic structural view of the guide plate provided by an embodiment of the present invention, as shown in Figure 2, the guide plate (3) extends along the length direction perpendicular to the guide plate. The guide plate 3 is provided with a first through hole 31 that is adapted to the guide tube unit. The first through hole 31 is of an elongated shape and extends along the length direction of the guide plate 3. The inlet end of the guide tube unit is inserted into the first through hole 31, and the guide tube unit and the guide plate 3 are fixedly connected.

The length of the main guide tube 1 is greater than that of the accessory guide tube 2, and the outlet end of the accessory guide tube 2 is attached to the outer side wall of the main guide tube 1.

In this embodiment, because the center line of the main guide tube 1 is not parallel with the center line of the accessory guide tube 2, such that the inlet end of the main guide tube and the inlet end of the accessory guide tube are not adjacent, and the two inlet ends are separated by a certain distance, this can prevent the insertion of larger device in the main guide tube 1 from affecting the device inserted into the accessory guide tube 2.

An endoscope view hole 4 is provided at the attachment site of the outlet end of the accessory guide tube 2 on the outer side wall of the main guide tube 1. As shown in Figure 1, the endoscope view hole 4 is an elongated hole and extends along the length direction of the accessory guide tube 2. The upper end opening of the endoscope view hole 4 is provided at the attachment site of the outlet end of the accessory guide tube 2 on the outer side wall of the main guide tube 1, the lower end opening of the endoscope view hole 4 is not in communication with the outlet end of the main guide tube 1.

Figure 3 is a second schematic diagram of the axial side structure of a working cannula provided by an embodiment of the present invention. As shown in Figure 3, in other embodiments, the lower end opening of the endoscope view hole 4 extends to the outlet end of the main guide tube 1, this can increase the length of the opening of the endoscope view hole 4, and the working state of the device in the accessory guide tube 2 can be more easily observed.

In order to facilitate a better insertion of the guide tube unit into the surgical site of the human body, the outlet end of the main guide tube 1 is provided with a first inclined opening 5, and the outlet end of the accessory guide tube 2 is provided with a second inclined opening 6. The inclination directions of the first inclined opening 5 and the second inclined opening 6 are the same. In this embodiment, the diameters of the main guide tube 1 and the accessory guide tube 2 are different, the diameter of the main guide tube 1 is larger than that of the accessory guide tube 2. The main guide tube 1 can be used to place a larger surgical device, and the accessory guide tube 2 can be used to place a smaller surgical device. Of course, in other embodiments, the diameter of the main guide tube 1 may also be smaller than that of the accessory guide tube 2, or the diameters of the main guide tube 1 and the accessory guide tube 2 are set to the same diameter. The multi-channel working cannula in the embodiments of the present invention has been described in detail above. The principle and embodiments of the present invention are set forth in this section by use of specific examples. The description of the above example is only used to facilitate the understanding of the core idea of the present invention.

The scope of protection of the present invention is defined by the claims.

## Claims

1. A multi-channel working cannula,
wherein the cannula includes a guide tube unit including a main guide tube (1) and an accessory guide tube (2) with the main guide tube (1) and the accessory guide tube (2) integrally arranged;
wherein one end of the guide tube unit is the inlet end, and the other end is the outlet end
wherein the inlet end of the guide tube unit is provided with a guide plate (3), the guide plate (3) being provided with a first through hole (31) that is adapted to the guide tube unit, the inlet end of the guide tube unit being inserted into the first through hole (31), and the guide tube unit being fixedly connected to the guide plate (31); wherein
the length of the main guide tube (1) is greater than that of the accessory guide tube (2), and an outlet end of the accessory guide tube (2) is attached to an outer side wall of the main guide tube (1) at an attachment site;
the multi-channel working cannula being **characterized in that,**
an endoscope view hole (4) is provided through the outer side wall of the main guide tube (1), at the attachment site of the outlet end of the accessory guide tube (2), such that an instrument in the main guide tube (1) can be more easily observed through the accessory guide tube (2);
wherein the endoscope view hole (4) is an elongated hole and extends along the length direction of the accessory guide tube (2).

2. The multi-channel working cannula according to claim 1, **characterized in that**:
an upper end of the elongated hole of the of the endoscope view hole (4) is provided at the attachment site of the outlet end of the accessory guide tube (2) on the outer side wall of the main guide tube (1), a lower end of the elongated hole of the of the endoscope view hole (4) is not in communication with an outlet end of the main guide tube (1).

3. The multi-channel working cannula according to claim 1, **characterized in that**:
a lower end of the endoscope view hole (4) extends to an outlet end of the main guide tube (1).

4. The multi-channel working cannula according to claim 1, **characterized in that**:
a center line of the main guide tube (1) is not parallel with a center line of the accessory guide tube (2).

5. The multi-channel working cannula according to claim 1, **characterized in that**:
an outlet end of the main guide tube (1) is provided with a first inclined opening (5).

6. The multi-channel working cannula according to claim 1, **characterized in that**:
the outlet end of the accessory guide tube (2) is provided with a second inclined opening (6).

7. The multi-channel working cannula according to claim 1, **characterized in that**:
the diameters of the main guide tube (1) and the accessory guide tube (2) are different.

8. The multi-channel working cannula according to claim 1, **characterized in that**:
the guide tube unit is made of stainless steel.

## Patentansprüche

1. Mehrkanal-Arbeitskanüle,
wobei die Kanüle eine Führungsrohreinheit enthält, die ein Hauptführungsrohr (1) und ein Führungsbegleitrohr (2) enthält, wobei das Hauptführungsrohr (1) und das Führungsbegleitrohr (2) einstückig angeordnet sind;
wobei ein Ende der Führungsrohreinheit das Einlassende ist, und wobei das andere Ende das Auslassende ist;
wobei das Einlassende der Führungsrohreinheit mit einer Führungsplatte (3) versehen ist, wobei die Führungsplatte (3) mit einem ersten Durchgangsloch (31) versehen ist, das an die Führungsrohreinheit angepasst ist, wobei das Einlassende der Führungsrohreinheit in das erste Durchgangsloch (31) eingesetzt ist, und wobei die Führungsrohreinheit mit der Führungsplatte (3) fest verbunden ist;
wobei die Länge des Hauptführungsrohrs (1) größer als das Führungsbegleitrohr (2) ist, und wobei ein Auslassende des Führungsbegleitrohrs (2) mit einer Außenseitenwand des Hauptführungsrohrs (1) an einem Befestigungsort befestigt ist;
wobei die Mehrkanal-Arbeitskanüle **dadurch gekennzeichnet ist, dass** ein Endoskopsichtloch (4) durch die Außenseitenwand des Hauptführungsrohrs (1) an dem Befestigungsort des Auslassendes des Führungsbegleitrohrs (2) derart bereitgestellt ist, dass ein Instrument in dem Hauptführungsrohr (1) einfacher durch das Führungsbegleitrohr (2) beobachtet werden kann;
wobei das Endoskopsichtloch (4) ein langgestrecktes Loch ist und sich entlang der Längsrichtung des Führungsbegleitrohrs (2) erstreckt.

2. Mehrkanal-Arbeitskanüle nach Anspruch 1, **dadurch gekennzeichnet, dass**:
ein oberes Ende des langgestreckten Lochs des Endoskopsichtlochs (4) an dem Befestigungsort des Auslassendes des Führungsbegleitrohrs (2) auf der Außenseitenwand des Hauptführungsrohrs (1) bereitgestellt ist, wobei ein unteres Ende des langgestreckten Lochs des Endoskopsichtlochs (4) in keiner Kommunikationsverbindung mit einem Auslassende des Hauptführungsrohrs (1) steht.

3. Mehrkanal-Arbeitskanüle nach Anspruch 1, **dadurch gekennzeichnet, dass**:
sich ein unteres Ende des Endoskopsichtlochs (4) bis zu einem Auslassende des Hauptführungsrohrs (1) erstreckt.

4. Mehrkanal-Arbeitskanüle nach Anspruch 1, **dadurch gekennzeichnet, dass**:
die Mittellinie des Hauptführungsrohrs (1) nicht parallel zu einer Mittellinie des Führungsbegleitrohrs (2) ist.

5. Mehrkanal-Arbeitskanüle nach Anspruch 1, **dadurch gekennzeichnet, dass**:
ein Auslassende des Hauptführungsrohrs (1) mit einer ersten geneigten Öffnung (5) versehen ist.

6. Mehrkanal-Arbeitskanüle nach Anspruch 1, **dadurch gekennzeichnet, dass**:
das Auslassende des Führungsbegleitrohrs (2) mit einer zweiten geneigten Öffnung (6) versehen ist.

7. Mehrkanal-Arbeitskanüle nach Anspruch 1, **dadurch gekennzeichnet, dass**:
die Durchmesser des Hauptführungsrohrs (1) und des Führungsbegleitrohrs (2) verschieden sind.

8. Mehrkanal-Arbeitskanüle nach Anspruch 1, **dadurch gekennzeichnet, dass**:
die Führungsrohreinheit aus rostfreiem Stahl hergestellt ist.

## Revendications

1. Canule fonctionnelle multicanaux,
la canule incluant une unité de tube de guidage incluant un tube de guidage principal (1) est un tube de guidage accessoire (2), le tube de guidage principal (1) et le tube de guidage accessoire (2) étant conçus de manière à être monoblocs ;
une extrémité de l'unité de tube de guidage étant l'extrémité d'entrée, et l'autre extrémité étant l'extrémité de sortie ;
l'extrémité d'entrée de l'unité de tube de guidage étant pourvue d'une plaque de guidage (3), la plaque de guidage (3) étant pourvue d'un premier trou traversant (31) qui est adapté à l'unité de tube de guidage, l'extrémité d'entrée de l'unité de tube de guidage étant insérée dans le premier trou traversant (31) et l'unité de tube de guidage étant connectée fixement à la plaque de guidage (3) ;
la longueur du tube de guidage principal (1) étant supérieure à celle du tube de guidage accessoire (2) et une extrémité de sortie du tube de guidage accessoire (2) étant fixée à une paroi latérale extérieure du tube de guidage principal (1) à un emplacement de rattachement ;
la canule fonctionnelle multicanaux étant **caractérisée en ce qu'**un trou de vision d'endoscope (4) est pratiqué à travers la paroi latérale extérieure du tube de guidage principal (1) à l'emplacement de rattachement de l'extrémité de sortie du tube de guidage accessoire (2), de sorte qu'un instrument se trouvant dans le tube de guidage principal (1) peut être observé plus facilement à travers le tube de guidage accessoire (2) ;
le trou de vision d'endoscope (4) étant un trou allongé et s'étendant dans le sens de la longueur du tube de guidage accessoire (2).

2. Canule fonctionnelle multicanaux selon la revendication 1, **caractérisée en ce qu'**une extrémité supérieure du trou allongé du trou de vision d'endoscope (4) est prévue à l'emplacement de rattachement de l'extrémité de sortie du tube de guidage accessoire (2) sur la paroi latérale extérieure du tube de guidage principal (1), une extrémité inférieure du trou allongé du trou de vision d'endoscope (4) n'étant pas en communication avec une extrémité de sortie du tube de guidage principal (1).

3. Canule fonctionnelle multicanaux selon la revendication 1, **caractérisée en ce qu'**une extrémité inférieure du trou de vision d'endoscope (4) s'étend jusqu'à une extrémité de sortie du tube de guidage principal (1).

4. Canule fonctionnelle multicanaux selon la revendication 1, **caractérisée en ce qu'**une ligne centrale du tube de guidage principal (1) n'est pas parallèle à une ligne centrale du tube de guidage accessoire (2).

5. Canule fonctionnelle multicanaux selon la revendication 1, **caractérisée en ce qu'**une extrémité de sortie du tube de guidage principal (1) est pourvue d'une première ouverture inclinée (5).

6. Canule fonctionnelle multicanaux selon la revendication 1, **caractérisée en ce que** l'extrémité de sortie du tube de guidage accessoire (2) est pourvue d'une seconde ouverture inclinée (6).

7. Canule fonctionnelle multicanaux selon la revendication 1, **caractérisée en ce que** les diamètres du tube de guidage principal (1) et du tube de guidage accessoire (2) sont différents.

8. Canule fonctionnelle multicanaux selon la revendication 1, **caractérisée en ce que** l'unité de tube de guidage est composée d'acier inoxydable.
